# EUROPEAN PATENT SPECIFICATION

(11) **EP 2 732 285 B1**
(45) Date of publication and mention of the grant of the patent: **09.08.2017**
(21) Application number: 12716262.6
(22) Date of filing: 24.04.2012
(51) Int. Cl.: G01N 33/50

(54) **SCREENING METHODS BASED ON VESICLE MOBILITY**
SCREENINGVERFAHREN AUF BASIS EINER VESIKELMOBILITÄT
PROCÉDÉ DE CRIBLAGE BASÉ SUR LA MOBILITÉ DES VÉSICULES

(30) Priority: 11.07.2011 SI 201100250 P
(43) Date of publication of application: 21.05.2014
(73) Proprietor: Celica, D.O.O., 1000 Ljubljana (SI); Centre of Excellence for Integrated Approaches in Chemistry and Biology of Proteins (CIPKeBiP), 1000 Ljubljana (SI)
(72) Inventor: ZOREC, Robert, 1000 Ljubljana (SI); STENOVEC, Matjaz, 1000 Ljubljana (SI); TRKOV, Sasa, 1000 Ljubljana (SI); VARDJAN, Nina, 1000 Ljubljana (SI); POTOKAR, Maja, 1291 Skofljica (SI); KREFT, Marko, 1000 Ljubljana (SI); GABRIJEL, Mateja, 8000 Novo mesto (SI); JORGACEVSKI, Jernej, 1000 Ljubljana (SI)
(74) Representative: Zacco GmbH
(86) International application number: PCT/EP2012/001759
(87) International publication number: WO 2013/007325

(56) References cited:
- WO-A1-2009/034094
- WO-A2-2007/067809
- POTOKAR MAJA ET AL: "Intermediate Filaments Attenuate Stimulation-Dependent Mobility of Endosomes/Lysosomes in Astrocytes", GLIA, vol. 58, no. 10, August 2010 (2010-08), pages 1208-1219, XP002682117, ISSN: 0894-1491
- TRKOV SASA ET AL: "Fingolimod-A sphingosine-like molecule inhibits vesicle mobility and secretion in astrocytes.", GLIA SEP 2012 LNKD- DOI:10.1002/GLIA.22361 PUBMED:22639011, vol. 60, no. 9, 25 May 2012 (2012-05-25), pages 1406-1416, XP002682118, ISSN: 1098-1136
- STENOVEC M ET AL: "Amyotrophic lateral sclerosis immunoglobulins G enhance the mobility of Lysotracker-labelled vesicles in cultured rat astrocytes", ACTA PHYSIOLOGICA, vol. 203, no. 4, December 2011 (2011-12), pages 457-471 URL, XP002682119,
- CHUN JEROLD ET AL: "Mechanism of Action of Oral Fingolimod (FTY720) in Multiple Sclerosis", CLINICAL NEUROPHARMACOLOGY, vol. 33, no. 2, March 2010 (2010-03), pages 91-101, ISSN: 0362-5664

## Description

### FIELD OF THE INVENTION

The present invention relates to in vitro methods for screening for pharmaceutically active substances useful in the treatment of multiple sclerosis.

### BACKGROUND OF THE INVENTION

Eukaryotic cells are relatively large in comparison to prokaryotic cells, and unlike the latter they exhibit numerous subcellular organelles. Some of these, such as secretory organelles - vesicles, recycling vesicles, various types of endosomes and lysosomes and others, participate in various forms of cell-to-cell communication. These organelles are relatively large (typically 50 to 500 nm in diameter), thus their diffusion-mediated mobility inside the cells is limited. Hence, in eukaryotic cells mechanisms have evolved that support rapid trafficking of these organelles, mainly along the cytoskeleton. The intact organelle transport to the intracellular targets and to the plasma membrane, where the exocytotic release of the organelle content into the extracellular space occurs, is a prerequisite for the normal cell function and intercellular communication based on chemical signaling. Recent technological advancements in fluorescence microscopy, associated by developments of new fluorescent probes enabling specific organelle labeling, provided, for the first time, high-resolution spatio-temporal imagining of organelle dynamics.

Higher brain functions rely primarily on finely tuned communication between neurons and glial cells that is mediated by the regulated release of signaling molecules, commonly known as neuro- and gliotransmitters. Disruptions in the intracellular vesicle traffic may lead to severe and debilitating diseases like Parkinson's disease, Huntington's disease, multiple sclerosis, amyotrophic lateral sclerosis (ALS), etc.

Astrocytes, the most abundant glial cell type in the central nervous system (CNS), exhibit an amazingly complex regulation of organelle mobility, which was described over the period of the last decade (Potokar et al. 2011. Histol Histopathol 26(2):277-84). They are multifunctional cells, modulating synaptic plasticity, learning, memory and sleep. Astrocytic processes enwrap synaptic terminals and establish connections with the blood capillaries. Astrocytes display a form of excitability that is based on elevations in intracellular calcium concentration (Ca²⁺) and are capable of releasing a variety of signaling molecules (gliotransmitters), like glutamate, adenosine triphosphate (ATP), D-serine and peptides, which directly modulate synaptic transmission and regulate cerebral blood flow and appear to play a key role in generating epilepsy, sleep disorders and other neurological pathologies.

Furthermore, astrocytes can act as "nonprofessional" antigen presenting cells (APCs), which unlike professional APCs (e.g. dendritic cells, macrophages, B-cells) do not express cell surface MHC (major histocompatibility complex) class II molecules constitutively, but only upon exposure to cytokine interferon gamma (IFNγ). In general, APCs play a key role in immune response. They take up and process exogenous antigens and present them to the CD4 helper T-cells, resulting in the activation of T-cells. Processing of antigens by APCs occurs through the endocytic pathway. After the internalization of antigens by endocytosis, early endosomes gradually transform into late endosomal/lysosomal compartments where antigens are processed to peptides and loaded onto MHC class II molecules. Late endosomal/lysosomal compartments expressing peptide-MHC class II complexes, termed MHC class II compartments, are then delivered to the surface of APC for recognition by the T cell receptors on the CD4 helper T-cells.

In the CNS, IFNγ activated astrocytes were shown to be involved in antigen presentation and activation of CD4 helper T-cells in immune-mediated neurological disorders, such as multiple sclerosis and its animal model experimental autoimmune encephalomyelitis (EAE). *In vitro,* in primary astrocyte cultures in the absence of neurons, the expression of surface MHC class II and some co-stimulatory molecules could be easily induced by exposure of cells to IFNγ. IFNγ activated primary cultures of astrocytes may therefore serve as a cell system to study molecular basis of antigen presentation in "nonprofessional" antigen presenting cells.

Fingolimod (FTY720) is a known first-in-class orally bioavailable compound that has been recently introduced to treat multiple sclerosis (MS). The mechanism of action of this lipophylic substance is considered to involve phosphorylation to form fingolimod-phosphate, which resembles naturally occurring sphingosine 1-phosphate (S1P), which plays a key role in the immune system, regulating lymphocyte egress from lymphoid tissues, thereby reducing autoaggressive lymphocyte infiltration into the central nervous system (CNS).

Methods for the analysis of vesicle mobility in cells by confocal fluorescence microscopy are also generally known (Potokar et al. 2005. Vesicle mobility studied in cultured astrocytes. Biochem Biophys Res Commun 329:678-83; Potokar et al. 2008. Stimulation inhibits the mobility of recycling peptidergic vesicles in astrocytes. Glia 56:135-44).

Despite the considerable developments in the technology for monitoring of intracellular organelle traffic (such as vesicle mobility), this technology has never been used for screening for substances useful in the treatment of the pathophysiological states, such as neurodegenerative disorders, e.g., Parkinson's disease, Huntington's disease, amyotrophic lateral sclerosis, or neuro-inflammatory diseases.

Nevertheless, there exists a great need for further and improved screening methods for indentifying active substances useful in the treatment of such diseases.

Against the background, it is an object of the present invention to provide systems and methods for the screening for substances useful in the treatment of specific diseases, in particular of neurodegenerative disorders, such as Parkinson's disease, Huntington's disease, amyotrophic lateral sclerosis; or neuro-inflammatory disorders, such as multiple sclerosis, meningitis, spinal arachnoiditis, encephalitis, myelitis and encephalomyelitis. Multiple sclerosis is a preferred medical indication of the present invention.

### SUMMARY OF THE INVENTION

The invention is based on the previously unrecognized relationship between the mobility of certain cell organelles (in particular organelles of glial cells or astrocytes) and a range of specific neural and inflammatory disorders. For example, the inventors were the first to observe a dose dependent variation of vesicle mobility, dependent on the extracellular concentration of FTY720, a substance known to be useful in the treatment of multiple sclerosis.

Experiments conducted by the inventors revealed that sphingosineand FTY720, but not the membrane impermeable lipid analogs, dose-dependently attenuated vesicle mobility in the regions near the plasmalemma and deeper in the cytoplasm. Cell permeable sphingosine-like lipids, such as FTY720, thus affect vesicle mobility, in particular the long-range vesicle mobility. From this new and hitherto unrecognized mechanism - and from the fact that FTY720 has proven useful in the treatment of multiple sclerosis - the inventors concluded that compounds acting on organelle mobility, in particular the long-range mobility, in screening experiments according to the present invention may be likely candidates for substances useful in the treatment of neurodegenerative disorders and neuro-inflammatory disorders, such as multiple sclerosis.

Furthermore, the present inventors considered that antigen-presenting cells (APCs) are key players in the immune response. They take up and process exogenous antigens and present them to CD4 helper T-cells, resulting in antigen-specific T-cell activation. After endocytosis of antigens by APCs, early endosomes gradually transform into late endosomes/lysosomes, where antigens are processed to peptides and loaded onto MHC class II molecules. Late endosomes/lysosomes expressing peptide-MHC class II complexes are delivered to the cell surface for recognition by T-cell receptors on CD4 helper T-cells. In inflammation and immune-mediated diseases of the central nervous system, astrocytes exposed to interferon-γ (IFN-γ) can express major histocompatibility complex (MHC) class II molecules and antigens on their surface. The inventors have now found that the mobility, in particular the long-range mobility, of MHC class II-positive vesicles is enhanced in such conditions. Therefore, vesicle mobility contributes to the efficiency of antigen presentation and represents a new therapeutic target in inflammatory and immune-mediated diseases. From this, the inventors concluded that substances that modify the mobility of vesicles in screening methods of the present invention are potential candidates for being useful substances in the treatment of inflammatory and neuro-inflammatory disorders.

The invention relates to an in vitro method of screening for a compound useful in the treatment of multiple sclerosis, said method comprising: (i) providing a glial cell; (ii) staining at least one vesicle of said glial cell; (iii) contacting said glial cell with a test compound; (iv) recording the path of said stained vesicle in said glial cell by obtaining multiple images of said stained vesicle in said glial cell at distinct points in time; (v) determining a mobility parameter characterizing the mobility of said stained vesicle in said glial cell, and (vi) comparing said mobility parameter obtained in step (v) with a reference mobility parameter obtained by performing steps (i) to (v) with a mixture of FTY720 and FTY720-phosphate, wherein the result of said comparison in step (vi) is taken as a measure for the likelihood that said test compound is useful in the treatment of said disease. The mobility parameter is preferably determined from at least one measurement of a length *L* between two distinct points on said recorded path.

In other embodiments, the invention relates to methods of determining the usefulness of a compound as a medicament useful in the treatment of a neurodegenerative disorder or a neuro-inflammatory disorder, such as multiple sclerosis, comprising steps (i) to (vi) above.

The methods of the invention are *in vitro*.

In preferred embodiments of the invention, the vesicle to be stained is an astrocytic vesicle.

In preferred embodiments of the invention, the glial cell is an astrocyte. Preferably, the glial cell is a rat cell or a mouse cell.

Preferably, the vesicle is fluorescently stained. In one embodiment, the staining of the vesicle is by transfection of the cell to express a protein tagged with a fluorescent marker, or by organelle-specific loading of said vesicle with a fluorescent substance. In one preferred embodiment the cell is transfected with a plasmid encoding atrial natriuretic peptide tagged with emerald green fluorescent protein (ANP.emd). In other embodiments, the glial cell is transfected with the plasmid encoding vesicle glutamate transporter-1 fluorescently tagged with EGFP (VGLUT1-EGFP). In other embodiments the staining is accomplished by staining with LysoTracker Red DND-99 (Invitrogen).

In preferred embodiments of the invention, the length *L* is the length of the straight line between said two distinct points on said recorded path. The mobility parameter may thus be the maximum displacement (MD) of said stained vesicle along its recorded path (Wacker et al., J Cell Sci 110 (Pt 13):1453-63). In other preferred embodiments of the length *L* is the length of the recorded path of said stained vesicle between said two distinct points on said recorded path. The mobility parameter may thus be the track length (TL). In further embodiments, the mobility parameter is the step length (displacement of a vesicle within the two successive images), the average step length, the speed of movement (nm/s), the angle of MD or the directionality index (DI, the ratio of MD to TL).

In preferred embodiments, the multiple images are obtained by fluorescence microscopy, preferably by confocal fluorescence microscopy. The multiple images are preferably obtained at a rate of 0.01-300, 0.1-100, or 1-10 per second.

The path of the vesicle in the cell may be recorded in 2D (i.e., only in one cross sectional plane through the cell), or in three dimensions.

In preferred embodiments of the invention, the reference mobility parameter is obtained by performing steps (i) to (v) with a reference compound.

The reference compound is preferably a mixture of FTY720 and FTY720-phosphate at a molar ratio from 1:0.01 to 1:100, or 1:0.1 to 1:100, or 1:0.1 to 1:10.

It is further preferred that the contacting takes place at a temperature of from 10°C to 40°C.

In preferred embodiments of the invention, the recording of the path of the vesicle (step (iv)) is over a time period of from 10 seconds to 10 hours, preferably from 1 minute to 10 hours, most preferred 1 minute to 1 hour.

Preferably, the multiple images are acquired at a magnification which allows the recording of a path comprising at least two points which are at least 250, 500, or 1000 nanometers apart from each other in space. This allows for the recording and analysis of the "long-range" mobility of the vesicle.

### BRIEF DESCRIPTION OF THE DRAWINGS

Figure 1 shows a test cell of the present invention.
Figure 2 shows (A) a two-dimensional analysis of a fluorescent light signal according to the invention, (B) a path of a stained organelle according to the invention, and (C)-(F) the results of an analysis of vesicle mobility with test substance (Sph) and reference compound (Stand).
Figure 3 shows fluorescence microphotographs according to the invention.
Figure 4 shows the effects of ATP and IFNγ on vesicle mobility, analyzed with methods according to the invention.

### DETAILED DESCRIPTION OF THE INVENTION

In the present invention isolated cell cultures of astrocytes (or other cell types) are used for testing biologically active compounds for their suitability of acting as prophylactic or therapeutic agents in the treatment of multiple sclerosis.

One aspect of the invention relates to an in vitro screening method for the identification of substances useful in the treatment of multiple sclerosis.

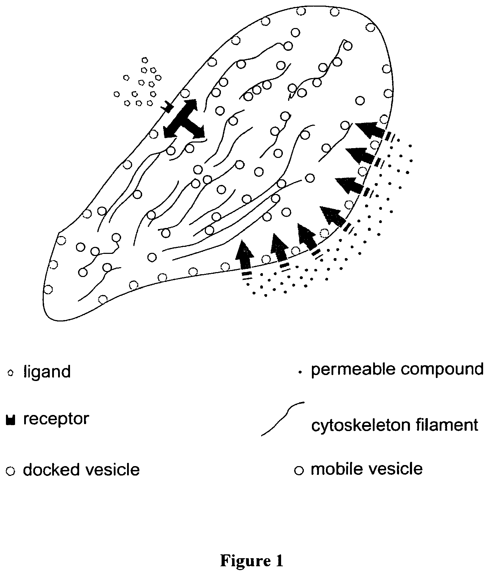

Figure 1 shows a schematic representation of the method developed for screening of compounds, e.g., serum and/or cerebrospinal fluid constituents for biological efficacy by analyzing their effect on single eukaryotic cells containing specifically labeled vesicles. The eukaryotic cell is treated either by a non-permeable ligand acting via plasma membrane receptor coupled to the intracellular messenger(s) pathway or permeable compound that freely crosses the plasma membrane and (in)directly affects trafficking of mobile vesicles along the cytoskeleton and/or probability for spontaneous or stimulated fusion of docked vesicles and the consequent release of their content into the extracellular space.

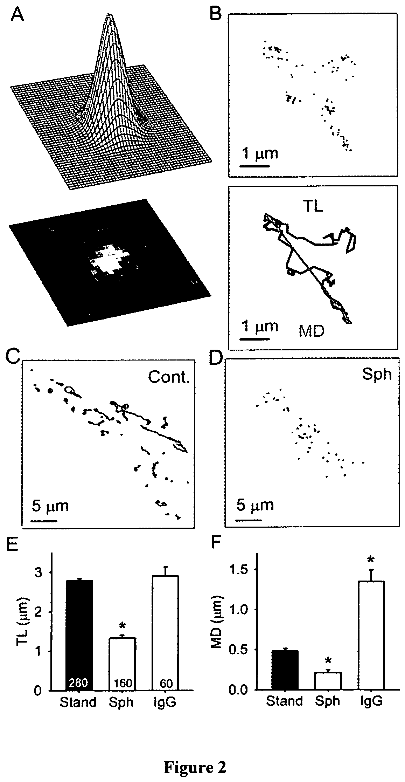

Figure 2 depicts an example of quantitative assessment of vesicle mobility used in the screening for biologically active substances which modulate vesicle mobility. In Fig. 2A is shown a 2D Gaussian curve (top) fitted to a fluorescence intensity signal of the selected vesicle (bottom) to obtain *x*, *y* coordinates of the vesicle position on the microscopic images. In Fig. 2B is shown the *x*, *y* coordinates (dots) of the peaks of the 2D Gaussian curve fitted to a single vesicle tracked over 120 s (top). The interconnected peaks of the curves represent the total vesicle path, from which the track length (TL) and the maximal displacement of a vesicle (MD) (bottom) can be calculated within a defined time epoch. In Fig. 2C are shown the vesicle tracks displaying vesicle movements in non-treated control astrocyte (Cont.) transfected with plasmid to express atrial natriuretic peptide tagged with emerald green fluorescent protein (ANP.emd) that is stored inside secretory vesicles. Many vesicles display directional mobility characterized by the elongated vesicle tracks. In Fig. 2D are shown vesicle tracks displaying vesicle movements in an astrocyte treated with sphingosine (Sph) which strongly attenuates vesicle mobility, which is indicated by the highly contorted vesicle tracks. In Fig. 2E is shown the mean (± s.e.m.) track length (TL), and in Fig. 2F is shown the mean (± s.e.m.) maximal displacement (MD) of vesicles in cells treated with the standard reference compound (Stand) and in cells treated with two different biologically active substances: 10 µM sphingosine (Sph), or 100 µg/ml human immunoglobulin G (IgG). Stimulus dependent differences in the vesicle mobility parameters are clearly seen. Sphingosine strongly attenuates both mobility parameters, while human IgG does not significantly affect the mean vesicle TL, however it increases the mean vesicle MD with respect to the standard compound. Numbers at the bar bases indicate the number of vesicles epochs (15 s) analyzed. *p<0.001 vs. standard.

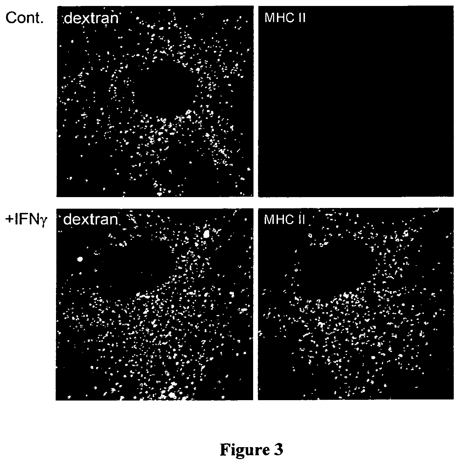

Figure 3 shows Alexa Fluor⁵⁴⁶ dextran labeled MHC class II positive compartments in IFNγ treated astrocytes. Fluorescence images of control (Cont.) and IFNγ treated (+IFNγ) astrocytes labeled by dextran, followed by fixation and immunostaining with antibodies against MHC class II molecules (MHC II). IFNγ treatment induces the punctuate expression of MHC class II molecules that overlay with dextran loaded structures. Dextran colocalizes with LAMP1, a marker for lysosomes (not shown).

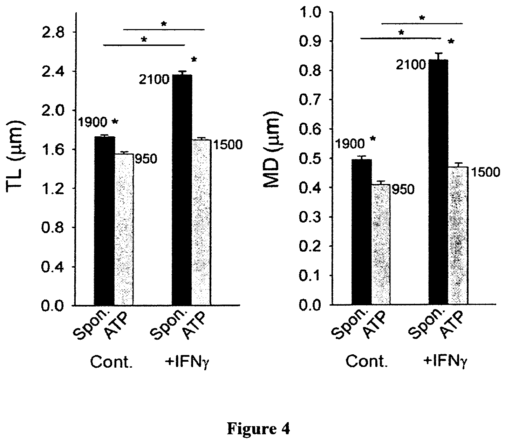

Figure 4 provides an example of the modulation of vesicle mobility associated with antigen presentation. In IFNγ treated astrocytes (+IFNγ) average vesicle track length and maximal displacement (± s.e.m.) significantly increased in comparison to control (Cont.) cells (Spon. bars). Cell stimulation with 1 mM adenosine triphosphate (ATP) reduced both mobility parameters in control and IFNγ treated cells. *p<0.05.

The invention is now further illustrated with reference to the following non-limiting examples.

### EXAMPLES

### Example 1

Cultured astrocytes, e.g. isolated as previously described by Schwartz & Wilson (Glia. 1992; 5(1):75-80) or any other cell type isolated from animal tissues or clonal cell lines derived from human or animal sources are seeded onto coverslips and maintained in an adequate cell cultured medium.

Cell organelles are stained by commercially available dyes and/or by cell transfection. E.g. peptidergic secretory vesicles are labeled by cell transfection for the secretory peptide - pro-atrial natriuretic peptide tagged with emerald green fluorescent protein (ANP.emd; see Han et al., Proc Natl Acad Sci USA. 1999; 96:14577-14582; Kražn et al., J Neurosci. 2003; 23(5):1580-3.). Glutamatergic vesicles are stained by cell transfection for vesicular glutamate transporter 1 tagged with green fluorescent protein (VGLUT1-GFP). ATP containing vesicles are stained by incubation of cells in quinacrine dihydrochloride (1 µM, 15 min at room temperature) (Pangrsic et al., J Biol Chem. 2007; 28;282(39):28749-58) and endosomes/ lysosomes by incubation of cells in LysoTracker Red DND-99 (200 nM, 5 min, RT, Invitrogen) (Potokar et al., Glia. 2010; 58(10):1208-19). To observe MHC class II expression cells are loaded with 0.1 mg/ml Alexa Fluor⁵⁴⁶ dextran (Molecular Probes, Invitrogen) for 16 hours at 37°C.

Cell cultures are treated with the biologically active substances (i.e. constituents of sera, cerebrospinal fluid and other biologically relevant small inorganic and organic substances, other related substances and mixtures of these), or with the reference standard compound, which consists of FTY720 and FTY720-phosphate (FTY720-P) in ratio from 1:0.1 to 1:100, dissolved in cell culture medium for 10 min at 18-37°C. To observe augmentation of the MHC class II molecule expression, cells are treated with interferon gamma (IFN-γ) for 48 hours at 37°C or any other stimulant of the antigen presentation.

Cell loaded coverslips are then rinsed with extracellular solution (consisting of 10 mM HEPES/NaOH (pH 7.2), 10 mM D-glucose, 130 mM NaCl, 2 mM CaCl₂, 2 mM MgCl₂, and 5 mM KCl) and observed by fluorescence microscopy at 63 × or 100 × magnification using 488 nm Ar-Ion laser excitation (for ANP.emd, VGLUT1-GFP and quinacrine dihydrochloride) or 543 nm He-Ne laser exitation (for LysoTracker Red DND-99, Alexa Fluor⁵⁴⁶ dextran), and band-pass filtered at 505-530 nm or long-pass filtered at 560 nm. Time-lapse fluorescence images are acquired at 0.5-2 Hz for 1-10 minutes to monitor vesicle mobility.

Cells can be stimulated with adenosine triphosphate (ATP) or any other stimulus of exocytotic release to record vesicle mobility and secretion before and after cell stimulation.

Vesicle traffic is analyzed by particle tracking software previously described (Potokar et al.; Biochem Biophys Res Commun. 2005; 329(2):678-83).

Several mobility parameters are determined for variable time epochs typically lasting 10-60 seconds: step length (displacement of a vesicle within the two successive frames), speed, track length (TL; the total path traveled by a vesicle within the defined time epoch), the vesicle maximal displacement (MD; a measure for the net translocation of a vesicle within the defined time epoch; cf. Wacker et al., J Cell Sci 110 (Pt 13):1453-63), the angle of MD and the directionality index (DI; the ratio of MD to TL).

In addition, cells can be fixed with 2-5% paraformaldehyde and immunostained with commercially available antibodies for the MHC class II to confirm augmented antigen presentation upon treatment with IFNγ or any other stimulant of the MHC class II expression, and observed by microscopy.

### Example 2

Example of the analysis of biologically active substances, such as sphingosine and immunoglobulin G (IgG) from human serum, on the mobility of secretory vesicles with respect to the effect of the standard compound, which is also the subject of the present invention. The example is shown in Figures 2E and F. The mobility of fluorescently labeled vesicles in transfected astrocytes expressing ANP.emd was analyzed by particle analyzing program Track2 and quantitative parameters of vesicle mobility were determined. The results demonstrate, that the cell treatment with 10 µM sphingosine (Sph) significantly attenuates vesicle mobility according to both mobility parameters - the average vesicle track length (TL) (Figure 2E) and the average maximal displacement (MD) (Figure 2F), while the human IgG does not have a significant effect on the average vesicle TL, however it increases the average MD with respect to the standard (Stand).

### Example 3

The example of vesicle mobility modulation in relation to the antigen presentation in IFNγ activated astrocytes. The example is demonstrated in Figures 3 and 4. As shown in Figure 3, MHC class II molecule loaded compartments co-localize with Alexa Fluor⁵⁴⁶ dextran loaded organelles. Therefore the mobility analysis of dextran-labeled vesicles can be applied to describe the modulated mobility of the vesicles carrying MHC class II molecules upon stimulation of antigen presentation in astrocytes. As shown in Figure 4, in IFNγ-treated astrocytes the average track length and the maximal displacement significantly increase in comparison with non-treated control cells. In contrast, cell stimulation with adenosine triphosphate (ATP) reduces vesicle mobility parameters in control as well as in activated cells.

### Example 4

This example describes an experimental procedure which can be applied in the screening methods according to the invention.

Primary astrocyte cultures were prepared from the cortices of 3-day-old Wistar rats. The care for experimental animals was in accordance with International Guiding Principles for Biomedical Research Involving Animals developed by the Council for International Organizations of Medical Sciences and Directive on Conditions for issue of License for Animal Experiments for Scientific Research Purposes (Official Gazette of the RS, No.40/85 and 22/87). Cells were grown in high-glucose DMEM containing 10% fetal bovine serum (Biochrom AG), 1 mM sodium pyruvate, 2 mM L-glutamine, and 25 µg/ml penicillin/streptomycin at 37°C, 5% CO₂ and 95% air atmosphere. Sub-confluent cultures were shaken at 225 rpm overnight with the subsequent medium change, which was repeated three times. Prior to experiments, the cells were trypsinized, sub-cultured onto poly-L-lysine-coated coverslips at 37°C, 5% CO₂ and 95% air atmosphere. In separate subsets of experiments, the cultured rat lactotrophs and mouse embryonic fibroblast - adipose like 3T3-L1 cells were prepared as described before (Kovacic et al., J Biol Chem 286:13370-81; Stenovec et al. 2004, FASEB J 18:1270-2). Unless stated otherwise, all chemicals were purchased from Sigma-Aldrich and were of highest purity grade available.

Cells were transfected with the plasmid encoding atrial natriuretic peptide tagged with emerald green fluorescent protein (ANP.emd; a gift from Dr. Ed Levitan, University of Pittsburgh, Pittsburgh, PA, USA) by nucleofection, using Rat Astrocyte Nucleofector Kit (Lonza) according to the manufacturer's instructions. Briefly, ~2 x 10⁶ cells were suspended in 100 µl of Rat Astrocyte Nucleofector Solution to which 2-5 µg of highly purified plasmid DNA was added. The cell suspension was transferred into a cuvette and exposed to nucleofection by the recommended pulse protocol in Nucleofector II (Amaxa Biosystems). The cell pellet was re-suspended in the culture medium and sub-cultured onto round 22 mm diameter poly-L-lysine-coated coverslips. The cultures were maintained at 37°C, 5% CO₂ and 95% air atmosphere and observed after 40-72 hours. Alternatively, astrocytes were transfected with the plasmid encoding vesicle glutamate transporter-1 fluorescently tagged with EGFP (VGLUT1-EGFP; kindly provided by Dr. Salah El Mestikawy, INSERM U513, Creteil Cedex, France) by nucleofection as described above. Acidic endosomes/lysosomes were stained by incubating astrocytes in the cell culture medium containing 200 nM LysoTracker Red DND-99 (Invitrogen) for 5 minutes at 37°C.

To observe the mobility of ANP.emd, VGLUT1-EGFP and LysoTracker labeled vesicles, astrocyte-loaded coverslips were washed 2x with extracellular solution containing 130 mM NaCl, 5 mM KCl, 2 mM CaCl₂, 1 mM MgCl₂, 10 mM D-glucose and 10 mM HEPES, pH 7.2, mounted into the recording chamber and transferred to a confocal microscope (LSM 510 & 780, Zeiss). The cells were observed by a plan-apochromatic oil-immersion objective 63×/NA 1.4. ANP.emd and VGLUT1-EGFP were excited by 488 nm argon laser line and emission fluorescence was filtered with 505-530 nm band-pass filter. LysoTracker Red DND-99 was excited with 543 nm He-Ne laser line and emission fluorescence filtered with long pass 560 nm filter. In initial experiments, the astrocyte plasma membrane was stained with red fluorescent styryl dye FM 4-64 (2 µM, Molecular Probes, Invitrogen) dissolved in the extracellular solution, to visualize the cell perimeter. FM 4-64 was excited by 488 nm argon laser line and emission fluorescence was filtered with 585 nm long-pass emission filter.

To study the mobility of fluorescently labeled vesicles, time-lapse images were acquired every 493 ms for 1-3 minutes in non-treated cells and cells treated with 1-20 µM FTY720 (Enzo Life Sciences), 10 µM sphingosine (Biomol) 10 µM FTY720-P (Echelon) or 10 µM thonzonium dissolved in the cell culture medium for 10 min at 37°C. Additionally, exocytotic stimulus composed of L-glutamic acid (L-Glu) and ATP (1 mM final concentration, each) was applied as a bolus into extracellular solution to all examined cells and the vesicle mobility recorded before and after stimulation.

In a separate set of experiments coverslips with ANP.emd transfected astrocytes were transferred into the recording chamber on the inverted microscope (Axio Observer.Z1, Zeiss) equipped with automated wheels for excitation filters and the beam-splitter/emission-filter cubes for epifluorescence imaging, and diode-pumped solid-state (DPSS) 488 nm laser (Toptica) for TIRF imaging. The emission fluorescence was collected with α-Plan-Apochromat oil-immersion objective 100×/NA 1.46 and alternate, dual mode (TIRF and epifluorescence) images were acquired with a CCD camera (AxioCamMRm, Zeiss) every 500 ms s for 1 minute prior to and 10 minutes after treatment with 10 µM FTY720. In epifluorescence mode ANP.emd was excited with 488 nm laser line and emission was band-pass filtered (DBP 525/31 + 616/57) using 74 HE filter set (Zeiss). In TIRF mode ANP.emd was excited with the evanescent field formed above the coverslip glass due to the total internal reflection of 488 nm laser beam (100 mW attenuated to 1-7%). Penetration depth (d) of the evanescent field was calculated by using the formula d = λ/4*π*(n₁²*sin²(α₁) - n₂²)^{-1/2}, where λ is the wavelength of illuminating light (488 nm), α₁ is the angle of incidence light, n₁ is the refractive index of the glass coverslips and the immersion oil (1.518), and n₂ is the refractive index of the cytosol (1.38). Depending on the angle of the incident light used for TIRF imaging, the calculated penetration depth was ∼150 nm.

To determine the time-course and the relative magnitude of the z-axis translocation of individual vesicles imaged in the TIRF plane, we considered the exponential dependence of the TIRF excitation intensity of the fluorescent objects positioned along the z-axis. Any change in the TIRF emission could result from: (1) changes in the vertical position of the fluorescent vesicle and/or (2) changes in the fluorescence of the vesicle itself (leak/discharge of the fluorophore, i.e., ANP.emd from vesicles, quenching/unquenching of the fluorophore, fluorophore bleaching). To assess the relative magnitude of the vesicle vertical displacement under epifluorescence illumination, we took advantage of the thin depth of field inherent to high numeric aperture objective used. The depth of field (d_{f}) was calculated by using the formula d_{f} = λ*n/NA² + (n/M*NA)*e, where λ is the wavelength of illuminating light (488 nm), n is the refractive index of immersion oil (1.518) between the coverslip and the objective front lens, NA is the objective numerical aperture (1.46), e is the smallest distance that can be resolved by a detector (sensor pixel size 6.45 x 6.45 µm) that is placed in the image plane of the microscope objective, whose lateral magnification is M (100). The displacement of a fluorescent vesicle below or above the objective depth of field inevitably resulted in images of unacceptable sharpness and decreased vesicle fluorescence due to diminished out-of-focus emission. To monitor individual vesicle fluorescence at distinct x-y positions over time, we manually centered the rectangular field over the vesicle in each sequentially acquired image and assessed the average fluorescence within the field by custom-written Matlab (Math Works, Natick, MA, USA) software t-TIME.

The mobility of peptidergic (ANP.emd), glutamatergic vesicles (VGLUT1-EGFP) and endosomes/lysosomes (LysoTracker labeled) was analyzed by ParticleTR software (Celica, Ljubljana, Slovenia) in exported tiff file images (Potokar et al., Biochem Biophys Res Commun 329:678-83). Briefly, a 2D Gaussian curve was fitted onto a selected vesicle in each image to obtain the x, y coordinates (peak of the curve), which were then connected to obtain the path that vesicles traveled within the total recording time. Typically, ~50 randomly selected vesicles were tracked per cell. The vesicle mobility parameters were estimated for 15 s epochs. For each vesicle the track length (TL - the path that individual vesicle traveled) and maximal displacement (MD - the farthest translocation of a vesicle) were determined. The analysis of the vesicle mobility was performed in transfected astrocytes, either not treated, acutely treated with FTY720 for 10 minutes, or pre-treated with 1-20 µM FTY720, 10 µM sphingosine, 10 µM FTY720-P or 10 µM thonzonium, and stimulated with 1 mM L-Glu and 1 mM ATP. The mean (±s.e.m.) vesicle TL and MD were determined in 9-15 cells treated by a particular compound.

The exocytotic cargo release from ANP.emd transfected astrocytes was determined in time-laps confocal images by two different approaches. Individual vesicle fusions with the plasma membrane followed by ANP release were identified as a sudden decrease in the vesicle fluorescence intensity indicating cargo discharge (Stenovec et al., 2004. FASEB J 18:1270-2). The time-resolved fluorescence intensity changes at the place of individual secreting vesicles were obtained by the LSM 510 and 780 software (Zeiss). The overall efficiency of vesicle cargo discharge following particular cell treatment was estimated by counting vesicles in exported tiff-format files using ImageJ software. The vesicles were counted on three consecutive confocal images taken before and 1 min after stimulation and the mean number of discharged vesicles determined relatively to their initial number. To test whether FTY720 alone stimulates exocytotic cargo discharge, the resting cells were observed for 1 minute and then treated with 20 µM FTY720 for the following 10 minutes. To test whether the cell pre-treatment with lipid compounds affects the vesicle cargo discharge, cells were pre-treated with 20 µM FTY720, 10 µM sphingosine or 10 µM thonzonium at 37°C for 10 min and subsequently exposed to the exocytotic stimulation by 1 mM L-Glu and 1 mM ATP. For statistical evaluation the vesicle numbers before and after stimulation were counted in 4-10 cells. The efficacy of exocytosis was expressed as the mean fraction of discharged vesicles after stimulation in non-treated and pre-treated cells.

The parameters of vesicle mobility, the cell and vesicle counts were expressed as the mean ± s.e.m. Statistical significance was determined with the two-tailed Student's t-test using SigmaPlot 11.0 (Systat Software Inc., USA).

## Claims

1. An in vitro method of screening for a compound useful in the treatment of multiple sclerosis, said method comprising:
(i) providing a glial cell;
(ii) staining at least one vesicle of said glial cell;
(iii) contacting said glial cell with a test compound;
(iv) recording the path of said stained vesicle in said glial cell by obtaining multiple images of said stained vesicle in said glial cell at distinct points in time;
(v) determining a mobility parameter characterizing the mobility of said stained vesicle in said glial cell from at least one measurement of a length *L* between two distinct positions on said recorded path, and
(vi) comparing said mobility parameter obtained in step (v) with a reference mobility parameter obtained by performing steps (i) to (v) with a mixture of FTY720 and FTY720-phosphate,
wherein the result of said comparison in step (vi) is taken as a measure for the likelihood that said test compound is useful in the treatment of said disease.

2. The method of claim 1, wherein said glial cell is an astrocyte.

3. The method of claim 1, wherein said staining is fluorescent staining by transfection of said cell with a plasmid encoding a fluorescently-tagged protein, or by organelle-specific loading of said vesicle with a fluorescent substance.

4. The method of any one of claims 1-3, wherein said length *L* is the length of the straight line between the two distinct positions on said recorded path.

5. The method of any one of claims 1-4, wherein said mobility parameter is a maximal displacement (MD).

6. The method of any one of claims 1-3, wherein said length *L* is the length of the recorded path of said stained vesicle between said two distinct points on said recorded path.

7. The method of any one of claims 1-3 or 6, wherein said mobility parameter is a track length (TL).

8. The method of any one of the preceding claims, wherein said multiple images are obtained by fluorescence microscopy, e.g., confocal fluorescence microscopy or TIRF microscopy.

9. The method of any one of the preceding claims, wherein said mixture of FTY720 and FTY720-phosphate is in a molar ratio from 1:0.1 to 1:100.

10. The method of any one of the preceding claims, wherein said recording of said path in step (iv) is over a time period of from 10 seconds to 10 hours.

11. The method of any one of the preceding claims, wherein said multiple images are at a magnification which allows the recording of a path comprising at least two positions which are at least 250 nanometers apart from each other in space.

## Patentansprüche

1. In-vitro-Verfahren zum Screenen auf eine zur Behandlung von multipler Sklerose nützliche Verbindung, wobei das Verfahren Folgendes umfasst:
(i) Bereitstellen einer Gliazelle;
(ii) Färben von zumindest einem Vesikel der Gliazelle;
(iii) Kontaktieren der Gliazelle mit einer Testverbindung;
(iv) Aufzeichnen des Weges des gefärbten Vesikels in der Gliazelle durch Erfassen von mehreren Bildern des gefärbten Vesikels in der Gliazelle zu verschiedenen Zeitpunkten;
(v) Bestimmen eines die Mobilität des gefärbten Vesikels in der Gliazelle charakterisierenden Mobilitätsparameters aus zumindest einer Messung einer Länge L zwischen zwei unterschiedlichen Position auf dem aufgezeichneten Weg, und
(vi) Vergleichen des in Schritt (v) erhaltenen Mobilitätsparameters mit einem Referenz-Mobilitätsparameter, der durch Durchführen der Schritte (i) bis (v) mit einem Gemisch aus FTY720 und FTY720-Phosphat erhalten wurde,
wobei das Ergebnis des Vergleichs aus Schritt (vi) als ein Maß für die Wahrscheinlichkeit, dass die Testverbindung zur Behandlung der Krankheit nützlich ist, herangezogen wird.

2. Verfahren nach Anspruch 1, wobei die Gliazelle ein Astrozyt ist.

3. Verfahren nach Anspruch 1, wobei das Färben fluoreszierendes Färben durch Transfektion der Zelle mit einem für ein fluoreszierend getaggtes Protein kodierenden Plasmid oder durch organellspezifisches Beladen des Vesikels mit einer fluoreszierenden Substanz ist.

4. Verfahren nach einem der Ansprüche 1-3, wobei die Länge L die Länge der Geraden zwischen den zwei unterschiedlichen Positionen auf dem aufgezeichneten Weg ist.

5. Verfahren nach einem der Ansprüche 1-4, wobei der Mobilitätsparameter eine maximale Verschiebung (MD) ist.

6. Verfahren nach einem der Ansprüche 1-3, wobei die Länge L die Länge des aufgezeichneten Wegs des gefärbten Vesikels zwischen zwei unterschiedlichen Punkten auf dem aufgezeichneten Weg ist.

7. Verfahren nach einem der Ansprüche 1-3 oder 6, wobei der Mobilitätsparameter eine Streckenlänge (TL) ist.

8. Verfahren nach einem der vorhergehenden Ansprüche, wobei die mehreren Bilder durch Fluoreszenzmikroskopie, z.B. konfokale Fluoreszenzmikroskopie oder TIRF-Mikroskopie, erfasst werden.

9. Verfahren nach einem der vorgehenden Ansprüche, wobei das Gemisch aus FTY720 und FTY720-Phosphat ein Molverhältnis von 1:0,1 bis 1:100 aufweist.

10. Verfahren nach einem der vorhergehenden Ansprüche, wobei das Aufzeichnen des Weges in Schritt (iv) über einen Zeitraum von 10 Sekunden bis 10 Stunden erfolgt.

11. Verfahren nach einem der vorhergehenden Ansprüche, wobei die mehreren Bilder eine Vergrößerung aufweisen, die das Aufzeichnen eines Weges zulässt, der mindestens zwei Positionen umfasst, welche räumlich um zumindest 250 Nanometer voneinander beabstandet sind.

## Revendications

1. Procédé in vitro de criblage d'un composé utile dans le traitement de la sclérose en plaques, ledit procédé comprenant:
(i) la fourniture d'une cellule gliale;
(ii) la coloration d'au moins une vésicule de ladite cellule gliale;
(iii) la mise en contact de ladite cellule gliale avec un composé d'essai;
(iv) l'enregistrement de la voie de ladite vésicule colorée dans ladite cellule gliale en obtenant des images multiples de ladite vésicule colorée dans ladite cellule gliale à des moments distincts dans le temps;
(v) la détermination d'un paramètre de mobilité caractérisant la mobilité de ladite vésicule colorée dans ladite cellule gliale à partir d'au moins une mesure d'une longueur *L* entre deux positions distinctes sur ladite voie enregistrée, et
(vi)la comparaison dudit paramètre de mobilité obtenu à l'étape (v) avec un paramètre de mobilité de référence obtenu par l'exécution des étapes (i) à (v) avec un mélange de FTY720 et de FTY720-phosphate,
dans lequel le résultat de ladite comparaison à l'étape (vi) est pris comme une mesure pour la probabilité de ce que ledit composé d'essai soit utile dans le traitement de ladite maladie.

2. Procédé selon la revendication 1, dans lequel la cellule gliale est un astrocyte.

3. Procédé selon la revendication 1, dans lequel ladite coloration est une coloration fluorescente par transfection de ladite cellule avec un plasmide codant pour une protéine marquée par fluorescence ou par chargement spécifique de l'organelle de ladite vésicule avec une substance fluorescente.

4. Procédé selon l'une quelconque des revendications 1 à 3, dans lequel ladite longueur L est la longueur de la ligne droite entre les deux positions distinctes de ladite voie enregistrée.

5. Procédé selon l'une quelconque des revendications 1 à 4, dans lequel ledit paramètre de mobilité est un déplacement maximal (MD).

6. Procédé selon l'une quelconque des revendications 1 à 3, dans lequel ladite longueur L est la longueur de ladite voie enregistrée de ladite vésicule colorée entre lesdits deux points distincts sur ladite voie enregistrée.

7. Procédé selon l'une quelconque des revendications 1 à 3 ou 6, dans lequel ledit paramètre de mobilité est une longueur de piste (TL).

8. Procédé selon l'une quelconque des revendications précédentes, dans lequel lesdites images multiples sont obtenues par microscopie à fluorescence, par exemple microscopie de fluorescence confocale ou microscopie TIRF.

9. Procédé selon l'une quelconque des revendications précédentes, dans lequel le mélange de FTY720 et de FTY720-phosphate est dans un rapport molaire de 1: 0,1 à 1: 100.

10. Procédé selon l'une quelconque des revendications précédentes, dans lequel ledit enregistrement de ladite voie dans l'étape (iv) est sur une période de temps comprise entre 10 secondes et 10 heures.

11. Procédé selon l'une quelconque des revendications précédentes, dans lequel lesdites images multiples sont à un agrandissement qui permet l'enregistrement d'une voie comprenant au moins deux positions qui sont espacées d'au moins 250 nanomètres l'une de l'autre dans l'espace.
